# EUROPÄISCHE PATENTSCHRIFT

(11) **EP 4 076 311 B1**
(45) Veröffentlichungstag und Bekanntmachung des Hinweises auf die Patenterteilung: **11.12.2024**
(21) Anmeldenummer: 20837935.4
(22) Anmeldetag: 14.12.2020
(51) Int. Cl.: A61F 13/00, A61F 13/02, A61F 13/06, A61F 13/08, D04B 21/16

(54) **KOMPRESSIONSBINDE**
COMPRESSION BANDAGE
BANDAGE COMPRESSIF

(30) Priorität: 17.12.2019 DE 102019134780
(43) Veröffentlichungstag der Anmeldung: 26.10.2022
(73) Patentinhaber: KOB GmbH, 67752 Wolfstein (DE)
(72) Erfinder: TAMOUÉ, Ferdinand, 67071 Ludwigshafen am Rhein (DE)
(74) Vertreter: DREISS Patentanwälte PartG mbB
(86) Internationale Anmeldenummer: PCT/EP2020/085886
(87) Internationale Veröffentlichungsnummer: WO 2021/122403

(56) Entgegenhaltungen:
- EP-A1- 3 078 360
- WO-A1-2010/085263
- WO-A1-2013/053410
- WO-A1-2017/109209
- DE-A1- 102007 063 294
- DE-A1- 102017 117 828
- US-A1- 2003 040 691
- US-A1- 2011 054 375

## Beschreibung

Die Erfindung betrifft eine Kompressionsbinde umfassend wenigstens zwei Vliesschichten.

Kompressionsbinden werden im Stand der Technik beispielsweise für diabetische Ulzera eingesetzt. Hierbei besteht das Problem, dass aufgrund des Krankheitsbildes notwendigerweise verschiedene Fragestellungen zu berücksichtigen sind, nämlich zum einen eine hinreichende Kompression, zum anderen aber auch eine Polsterwirkung gegenüber der zu behandelnden Gliedmaße.

So kann es beispielsweise vorgesehen sein, eine Polsterung aufzubringen und darüber eine Kompressionsbinde anzulegen.

Dabei sind bei der Kompressionstherapie grundsätzlich der sogenannte Arbeitsdruck und der sogenannte Ruhedruck zu unterscheiden, wobei der Ruhedruck der Druck ist, der bei liegender Gliedmaße durch das Kompressionsmittel, hier die Kompressionsbinde, auf die Gliedmaße ausgeübt wird. Der Arbeitsdruck ist dann der Druck, der auf die Gliedmaße bei Bewegung der Muskeln ausgeübt wird. Bevorzugt soll der Arbeitsdruck 20 bis 40 mm Hg über dem Ruhedruck liegen.

Es ist dabei ebenfalls bekannt, verschiedene Bindentypen einzusetzen. So sind sogenannte Langzugbinden bekannt, die eine sehr hohe Elastizität aufweisen und häufig eine Dehnfähigkeit über 200 % besitzen, wobei demgegenüber Kurzzugbinden bekannt sind, die nur eine geringe Dehnbarkeit besitzen und nur eine geringe Rückstellkraft, jedoch bereits sehr früh keine weitere Dehnung zulassen und so einen vergleichsweise hohen Arbeitsdruck aufzubauen vermögen. Demgegenüber bauen Kurzzugbinden über einen vergleichsweise langen Bereich nur einen geringen Widerstand auf, um dann eine sehr hohe Begrenzung der Dehnbarkeit zu realisieren. Die traditionelle Kurzzugbandagenkompressionstherapie ist eine Maßnahme für die Behandlung von Venenerkrankungen. Dabei werden die Materialien für Kurzzugbinden in der Regel aus nicht elastischen Materialien gefertigt und durch Ausrüstungsprozesse elastifiziert. Die Elastizität reduziert sich jedoch während der Behandlung signifikant. Dies kann zu einer Reduktion des Kompressionsdrucks in der Anwendung führen.

Bekannte Kompressionsbandagen sind beispielsweise in der EP 2 275 062 A2 beschrieben, die eine innere der Haut zugewandte elastische Bandage beschreibt mit einem gedehnten elastischen Substrat und einer gedehnten Schaumschicht, die auf der hautzugewandten Seite des Substrats angeordnet ist, sowie einer weiteren gedehnten selbstklebenden elastischen Bandage, die hierüber angelegt wird.

Weiterhin sind Kompressionsbinden aus mehreren miteinander vernähten Vlieslagen bekannt, z.B. aus der WO 2017/109209, die ggf. mit einer weiteren Bindenlage kombiniert werden können.

Zur Erreichen einer hohen therapeutischen Sicherheit ist es wünschenswert, dass der therapeutisch notwendige Kompressionsdruck möglichst gut erreicht wird. Hierzu sind im Stand der Technik eine Vielzahl von Möglichkeiten bekannt. So sind beispielsweise Markierungsmittel bekannt, die bei einer zu großen Dehnung eine Verformung erfahren, so dass der Therapeut eine zu große Dehnung und damit einen zu hohen Anlegedruck erkennen kann. Nachteilig ist jedoch dabei, dass hierfür stets geschultes Personal notwendig ist.

Darüber hinaus besteht bei der Behandlung von Ödemen oder chronischen Wunden oftmals neben dem Ziel die bestehenden Ödeme zu reduzieren und den beschleunigten Abtransport von Wundexsudat zu ermöglichen der Nachteil, dass das anfallende Wundexsudat insbesondere bei chronischen Wunden, bei denen solche Kompressionstherapien eingesetzt werden, dem Heilungsverlauf der Wunde entgegenwirkt. So enthält das Wundexsudat z.B. Enzyme, die den Aufbau einer extrazellulären Matrix verhindern. Derartige Enzyme sind z.B. MMP (Matrix-Metalloproteinasen). Es müssen daher zusätzliche Wundauflagen unterhalb der Kompressionsbinde angelegt werden. Hier besteht die Gefahr, dass die Wundauflage gegenüber der Kompressionsbinde verrutscht und daher ihre Wirkung nicht wie vorgesehen entfaltet.

Es ist daher wünschenswert, eine Kompressionsbinde, die alternativ auch als Kompressionsverband oder -bandage bezeichnet werden kann, bereitzustellen, die eine hohe Anlegesicherheit aufweist bei gleichzeitig guten therapeutischen Eigenschaften.

Sofern die Begriffe Schicht oder Lage verwendet werden, bezeichnen diese den gleichen Gegenstand.

Ausgehend von diesem Stand der Technik löst die Erfindung die Aufgabe durch eine Kompressionsbinde mit den Merkmalen des Anspruchs 1.

Dabei besteht die erste Vliesschicht aus einem Vliesmaterial, das superabsorbiere Fasern (SAF) beinhaltet. Eine solche Binde hat den Vorteil, dass die erste Vliesschicht zugleich neben der Tatsache, dass sie insbesondere über mehrere Tage getragen werden kann, ohne dass ein deutlicher Abfall des Kompressionsdrucks festzustellen ist, zusätzlich in der Lage sind, Wundflüssigkeiten aufzunehmen und zu binden. Es hat sich gezeigt, dass superabsorbierende Fasern Proteasen über diffusible Mechanismen inhibieren als auch durch direkte Bindung kompartmentieren und damit dem Wundexsudat bzw. der Wunde entziehen können. Insbesondere hat sich gezeigt, dass superabsorbierende Fasern zur Inhibition von Proteasen in chronischen Wunden geeignet sind. Zudem hat sich gezeigt, dass Metallo-Proteasen durch superabsorbierende Fasern gebunden oder kompartimentiert werden, so dass diese Metallo-Proteasen mit den superabsorbierenden Fasern aus einer Wundflüssigkeit oder einer Wunde entfernt werden können. Somit kann mittels der superabsorbierenden Fasern ein Überschuss an Metallo-Proteasen in chronischen Wunden derart abgefangen werden, dass ein natürlicher Heilungsverlauf stattfinden kann.

Die erste Vliesschicht kann dabei aus einen Vliesmaterial bestehen, in das oder auf das superabsorbierende Fasern eingebracht oder aufgebracht sind. Nach einer besonderen Ausführungsform kann das Vliesmaterial auch vollständig aus superabsorbierenden Fasern bestehen.

Die zweite Vliesschicht und die erste Vliesschicht sind mittels Nähwirkverfahren über einen elastischen Faden miteinander verbunden. Insbesondere beträgt die Stichlänge 1,5 bis 3 mm/u bei einer Nähfadenspannung von höchstens 4 cN. Die Verbindung erfolgt insbesondere in ungedehntem Zustand.

Durch diese Gestaltung kann erreicht werden, dass die mindestens beiden Schichten mittels des Nähwirkverfahrens und hier vorzugsweise einem Malimo- bzw. Maliwatt-Verfahren verbunden werden, wobei die Schichten vorzugsweise im ungedehnten Zustand mittels des elastischen Nähfadens verbunden werden.

Der Vorteil bei einem Nähwirkverfahren besteht darin, dass gleichzeitig an mehreren Stellen eine Verbindung durchgeführt werden kann und die beiden Schichten nach der Verbindung nicht mehr voneinander getrennt werden können. Über die Auswahl der Stichlänge in Längsrichtung des Flächengebildes aus dem dann die Kompressionsbinden konfektioniert werden, wobei unter Stichlänge der Abstand in Stichlängsrichtung zwischen zwei Stichen verstanden werden soll, und der Nähfadenspannung die Dehnbarkeit des elastischen Verbundes eingestellt werden kann, so dass ein von Hand nicht mehr trennbarer und trotzdem kontrollierbar elastischer Verbund aus den beiden Schichten besteht. Je nach Auswahl dieser Parameter zieht sich das fertige Flächengebilde zusammen bei Entspannung und werden Falten im Material aufgeworfen.

Die Nähtechnik und die Stichlänge des Nähfadens werden dabei bevorzugt so reguliert, dass die Fasern auf der ersten Vliesschicht auf der einen Seite des Verbundes aus den zwei Schichten Hautkomfort und Ausgleichsfunktionen besitzen und damit letztendlich die Kompressionsbinde insbesondere zwei erkennbar unterschiedliche Seiten aufweist, die für den Druckausgleich sehr funktional sind. Des Weiteren ist die Stichlänge insbesondere dabei so einzustellen, dass die gewünschten absorbierenden sowie hautfreundlichen Eigenschaften der der Haut zugewandten erste Vliesschicht erhalten bleiben.

Es sind jedoch auch andere Verbindungsarten denkbar wie zum Beispiel über eine Vernadelung, eine klebende Verbindung, eine thermische Verbindung, über Schweißstellen und/oder über Druck. Die Verbindung kann dabei flächig, auch partiell, oder linienförmig oder punktuell erfolgen.

Die erste Vliesschicht ist dabei vorzugsweise die einer Gliedmaße zugewandte Seite einer Kompressionsbinde und die zweite Vliesschicht die hierauf aufgebrachte zweite Seite. Es ist jedoch auch eine Anordnung in umgekehrter Weise denkbar, sofern die zweite Vliesschicht oder ggf. vorgesehene weitere Schichten den Durchtritt an Exsudat erlauben. Dazwischen, also zwischen erster und zweiter Vliesschicht, oder auf der der ersten Vliesschicht abgewandten Seite der zweiten Vliesschicht können weitere Schichten mit Polsterwirkung oder stabilisierenden oder haftenden Funktionen vorgesehen sein. Neben weiteren Vliesschichten können auch Folienschichten oder aus elastischen Fäden gebildeten Schichten vorgesehen sein. Die Schichten können dabei kontinuierlich oder diskontinuierlich sein und insbesondere vollflächig bezüglich der ersten und/oder zweiten Vliesschicht oder nur partiell.

Sofern ein Nähwirkverfahren vorgesehen ist, wird insbesondere das Malimo- bzw. Maliwattverfahren zum Verbinden der Schichten, wie im Stand der Technik bekannt eingesetzt. So kann z.B. die Elastizität durch Übernähen eines starren Vliessstoffes oder Gewebes mit dauerelastischen Elastanfäden in Längsrichtung unter Anwendung der Nähwirktechnik MALIWATT oder Malimo erhalten wurde. Die Nähwirktechnik MALIWATT bzw. Malimo ist in Malimo Nähwirktechnologie, Ploch, Böttcher, Scharch, VEB Fachbuchverlag Leipzig, 1978, 1. Auflage beschrieben.

Bevorzugt können eine oder beide Schichten, also die erste Vliesschicht und die zweite Vliesschicht unelastisch ausgebildet sein und werden erst elastifiziert, zum Beispiel durch das Nähwirkverfahren. Das gilt bevorzugt auch für weitere optionale Schichten. Alternativ kann die Elastifizierung auch durch das Einbringen oder Aufbringen von elastischen Fäden oder elastischen Folien erfolgen.

Weiter bevorzugt sind die erste und die zweite Vliesschicht bezüglich ihrer Erstreckungen in gleich. Insbesondere sind sie in Breitenrichtung kantenbündig ausgebildet. Die beiden Vliesschichten liegen flächig aufeinander auf.

Nach einem Ausführungsbeispiel kann es sich bei der ersten Vliesschicht um eine Wattevliesschicht, insbesondere eine Thermofusionsvliesschicht, handeln, die gegebenenfalls auch schon vorvernadelt sein kann. Dabei werden bei beiden Verfahren, nämlich dem Thermobonding als auch der der Thermofusion die Fasern der Vliese in einem Kämmereiverfahren in eine bestimmte Richtung gelegt und in einem Textilfunktionalisierungsverfahren als Vliesrollen vorbereitet und durch Temperatur oder durch Temperatur und Druck stabilisiert für die Weiterverarbeitung. Während des Thermofusionsverfahrens werden Fasern mit verschiedenen Schmelzpunkten durch Heißluft-Trockner miteinander verschmolzen. Die superabsorbierenden Fasern werden hierbei mit verarbeitet.

Im Thermobondingverfahren werden die Fasern mittels Hitze und Druck zwischen beheizten Kalanderrollen verschmolzen.

Das Ergebnis sind in beiden Fällen weiche, homogene Vliesstoffe, die ideal und für technische Anwendungen geeignet sind.

Das Thermofusionsverfahren eignet sich aufgrund des fehlenden Drucks besser für erste Vliesschichten, sofern diese eine Polsterfunktion aufweisen soll.

Bei der zweiten Vliesschicht kann es sich bevorzugt um ein Thermobondvlies handeln. Das Thermobondvlies weist dabei vorzugsweise lediglich eine geringe Dehnfähigkeit bei gleichzeitig gewünschter Steifigkeit auf. Als Grundmaterial des Vlieses für die erste und/oder zweite Vliesschicht kommt bevorzugt Polyester zum Einsatz. Als alternative Materialien kommen Viskose, Cellulose, Alginat, Acetat, Polyamid, Polyethylen, Polypropylen oder Kombinationen hiervon in Frage. Grundsätzlich kann die zweite Vliesschicht ebenfalls superabsorbierende Fasern aufweisen.

Bei den superabsorbierenden Fasern handelt es sich bevorzugt um Fasern aus einem organischen Material, das synthetisches und/ oder natürliches Material umfassen kann, wie beispielsweise Agar, Pektin und Guargummi oder auch synthetische Materialien, wie beispielsweise synthetische Hydrogelpolymere. Synthetische Hydrogelpolymere umfassen beispielsweise Carboxymethylcellulose, Alkalimetallsalze von Polyacrylsäuren, Alkalimetallsalze von Polymethacrylsäuren, Polyacrylamide, Polyvinylalkohol, Ethylenmaleinsaeureanhydrid-Copolymere, Polyvinylether, Hydroxypropylcellulose, Polymere und Copolymere von Vinyl- Sulfonsäure, Polyacrylate, Polymethacrylate, Polyacrylat-Polymethacrylat-Copolymere, Polyacrylamide und Ähnliche. Ganz besonders bevorzugt sind als superabsorbierende Fasern solche Fasern, die aus Alkalimetallsalzen von Polyacrylsäuren, Alkalimetallsalzen von Polymethacrylsäuren, Polyacrylaten, Polymethyacrylaten oder Polyacrylat-Polymethacrylat-Copolymeren hergestellt sind. Die Hydrogelpolymere sind vorzugsweise leicht vernetzt um die Materialien im Wesentlichen wasserunlöslich zu machen. Die Vernetzung kann z. B. durch Bestrahlung oder kovalente, ionische, von van-der-Waals- oder Wasserstoffbrückenbindung geschehen. Geeignete Materialien sind von verschiedenen Herstellern wie BASF und Stockhausen Inc. erhältlich.

Insbesondere ist hierbei vorgesehen, dass die zweite absorbierende Schicht als absorbierendes Material ein Faservlies umfasst, das ein Fasergemisch aus superabsorbierenden Fasern und Stützfasern umfasst. Hierbei kann jedoch auch vorgesehen sein, dass das Faservlies superabsorbierenden Fasern und Stützfasern umfasst und die Stützfasern ausgewählt werden aus der Gruppe der thermoplastischen Fasern und/ oder Cellulosefasern und/ oder Viskosefasern. Ganz besonders bevorzugt umfasst das Fasergemisch superabsorbierende Fasern, Viskosefasern und thermoplastische Fasern wobei als thermoplastische Fasern weiterhin bevorzugt Polypropylen- oder Polyethylenfasern verwendet werden können. Diese thermoplastischen Fasern können auch als so genannte Bikomponentenfasern in Form von Mantel-Kern-Fasern vorliegen.

Dabei enthält das zweite absorbierende Material vorzugsweise ein Fasergemisch, das mehr als 20 Gew.-%, insbesondere mehr als 30 Gew.-%, bevorzugt mehr als 40 Gew.-%, bevorzugt mehr als 50 Gew.-% und besonders bevorzugt mehr als 60 Gew.-% superabsorbierende Fasern umfasst. Hierdurch kann ein absorbierendes Kissen bereitgestellt werden, das eine besonders hohe freie Absorption aufweist. Eine entsprechend ausgerüstete Wundauflage kann bis zu drei und mehr Tagen auf einer Wunde verbleiben.

Die Bezeichnung "superabsorbierend" bezieht sich dabei auf ein in Wasser quellbares, im wesentlichen wasserunlösliches Material in Form von Fasern, das in der Lage ist, mindestens das ca. 10-fache, vorzugsweise ca. 20-fache und besonders bevorzugt ca. 50-fache oder mehr seines Gewichts an Wasser zu absorbieren. Das superabsorbierende Material kann dabei aus einem organischen Material gebildet sein, das synthetisches und/ oder natürliches Material umfassen kann, wie beispielsweise Agar, Pektin und Guargummi oder auch synthetische Materialien, wie beispielsweise synthetische Hydrogelpolymere. Synthetische Hydrogelpolymere umfassen beispielsweise Carboxymethylcellulose, Alkalimetallsalze von Polyacrylsäuren, Alkalimetallsalze von Polymethacrylsäuren, Polyacrylamide, Polyvinylalkohol, Ethylenmaleinsaeureanhydrid-Copolymere, Polyvinylether, Hydroxypropylcellulose, Polymere und Copolymere von VinylSulfonsäure, Polyacrylate, Polymethacrylate, Polyacrylat-Polymethacrylat-Copolymere, Polyacrylamide und Ähnliche. Ganz besonders bevorzugt sind als superabsorbierende Fasern solche Fasern, die aus Alkalimetallsalzen von Polyacrylsäuren, Alkalimetallsalzen von Polymethacrylsäuren, Polyacrylaten, Polymethyacrylaten oder Polyacrylat-Polymethacrylat-Copolymeren hergestellt sind. Die Hydrogelpolymere sind vorzugsweise leicht vernetzt um die Materialien im Wesentlichen wasserunlöslich zu machen. Die Vernetzung kann z. B. durch Bestrahlung oder kovalente, ionische, von van-der-Waals- oder Wasserstoffbrückenbindung geschehen. Geeignete Materialien sind von verschiedenen Herstellern wie BASF und Stockhausen Inc. erhältlich.

Weiterhin bevorzugt ist hierbei vorgesehen, dass die erste Vliesschicht als absorbierendes Material ein Faservlies umfasst, das ein Fasergemisch aus mindestens 20 Gew.-% superabsorbierenden Fasern, mindestens 10 Gew.-% Viskose- oder Cellulosefasern und mindestens 5 Gew.-% thermoplastische Fasern umfasst. Ganz besonders bevorzugt ist vorgesehen, dass die zweite absorbierende Schicht als absorbierendes Material ein Faservlies umfasst, das ein Fasergemisch aus mindestens 40 Gew.-% superabsorbierenden Fasern, mindestens 20 Gew.-% Viskose- oder Cellulosefasern und mindestens 5 Gew.-% thermoplastische Fasern umfasst. Durch den Anteil an Stützfasern in dem Faservlies erfolgt zu Beginn der Absorption eine gleichmäßige Verteilung der aufzunehmenden Flüssigkeitsmenge.

Die beiden Schichten sowie andere Schichten, sofern sie mittels Übernähen verbunden werden, werden zusammen einer Kettenwirkmaschine zugeführt und mittels eines elastischen Nähfadens, der vorzugsweise aus einer Gruppe aus Baumwollspinnkreppfäden, Baumwollzwirnkreppfäden, texturierten Polyamidgarnen, texturierten Polyestergarnen, Gummifäden oder Polyurethanelastanfäden oder einer Kombination hiervon ausgewählt werden kann, miteinander verbunden.

Der Nähfaden kann alternativ auch als Kettfaden bezeichnet sein. Der Faden verläuft dabei in Maschinenrichtung der Kettenwirkmaschine und nicht quer hierzu.

Das fertig konfektionierte, insbesondere vernähte Flächengebilde der Kompressionsbinde weist bevorzugt eine optimierte Dehnung auf, wobei besonders bevorzugt vorgesehen sein kann, dass die maximale Dehnbarkeit der Kompressionsbinde, der einer vorgegebenen optimalen Dehnbarkeit entspricht, und eine hierüber hinausgehende Dehnung der Kompressionsbinde durch eine Dehnungsschwelle begrenzt ist. Auf diese Weise kann die Anlegesicherheit signifikant erhöht werden, da es auch für nicht geübte Anwender möglich ist, die Kompressionsbinde maximal bis zur Dehnungsschwelle zu dehnen, wobei dann nicht nur die maximale Dehnbarkeit, sondern zugleich auch die optimale Dehnung und damit der optimale Kompressionsdruck erreicht ist und in diesem maximal gedehnten Zustand die Kompressionsbinde anzulegen. Die Dehnungsschwelle kann dabei auf verschiedene Weise eingestellt werden. So ist zum Beispiel über eine thermische Behandlung (u.a. Thermosetting) die Elastizität einstellbar.

Sofern die Binde als Polsterbinde dient, kann, um eine optimale Polsterwirkung zu erzielen, es bevorzugt vorgesehen sein, dass die Dicke der als Polsterschicht dienenden Vliesschicht 0,3-12 mm, bevorzugt 0,4-6 mm und weiter bevorzugt 0,5-3 mm, besonders bevorzugt 0,6-1,2mm beträgt.

Im Fall der bevorzugten Verbindung mittels eines Nähwirkverfahrens werden die beiden Schichten im entspannten Zustand, nachdem das vernähte Flächengebilde mittels Längskonfektionierung zur Kompressionsbinde weiterverarbeitet wurde, so in Wellen gelegt, dass eine unregelmäßige Oberfläche der Kompressionsbinde entsteht. Aufgrund dieser unregelmäßigen Oberfläche wird neben der primären Funktion als Ausgleichslage der Kompressionsbinde und der sekundären Funktion der regulierbaren Dehnbarkeit und damit erhöhten Anlegesicherheit auch erreicht, dass durch die Wellen ein Muster auf der Oberfläche entsteht aus Materialerhebungen und Materialvertiefungen, dass auch im maximal gedehnten Zustand nicht vollständig aufgehoben ist, so dass in der Therapie hierdurch zusätzlich ein Massage- bzw. Drainageeffekt entsteht.

Um die Anlagesicherheit weiter zu verbessern, kann vorgesehen sein, auf der dem Träger abgewandten Seite, insbesondere der der ersten Vliesschicht abgewandten Seite der Kompressionsbinde, insbesondere auf der zweiten Vliesschicht, eine kohäsive Beschichtung vorzusehen.

Eine mögliche Ausgestaltung der Kompressionsbinde kann dabei eine Binde aus zwei Vlieslagen aus Polyestermaterial mit superabsorbierenden Fasern in mindestens einer der beiden Lagen, wobei das zweilagige Grundtextil durch Übernähen eines starren Polyestervliesstoffs mit dauerelastischen Elastanfäden in Längsrichtung unter Anwendung der Nähwirktechnik MALIWATT erhalten wurde. Die Nähwirktechnik MALIWATT ist in Malimo Nähwirktechnologie, Ploch, Böttcher, Scharch, VEB Fachbuchverlag Leipzig, 1978, 1. Auflage beschrieben. Diese Kompressionsbinde ist auf einer Oberfläche mit einem kohäsiv haftenden Klebemittel beschichtet, um den gewünschten kohäsiven Hafteffekt zu erreichen.

Die Binde umfasst dabei folgende Zusammensetzung über beide Schichten:
65 % Polyester ,8 % Elastan, 7 % synthetischer Kautschuk, Flächengewicht gedehnt 105 +/- 20 g/qm nach DIN 61632, Dehnbarkeit / Rückzug nach DIN 61632 65 +/-20 %

Zur Aufbringung eines Ruhedrucks kann darüber hinaus vorgesehen sein, die erfindungsgemäße Kompressionsbinde mit einer weiteren Kompressionsbinde zu kombinieren, insbesondere zu überwickeln, die z.B. als Langzugbandage ausgebildet sein kann, d.h., von Beginn der Dehnung an einen Kompressionsdruck bereitzustellen, der jedoch nicht sprunghaft ansteigt.

Als weitere Binde für den Ruhedruck wird bevorzugt eine elastische, kohäsiv haftende Vliesbinde vom Typ 752, Handelsname NOWOPRESS 752, Hersteller: Karl Otto Braun GmbH & Co. KG, Wolfstein, Deutschland als Kompressionsbinde mit Langzugeigenschaften verwendet, wobei das Grundtextil durch Übernähen eines starren Polypropylenvliesstoffs mit dauerelastischen Elastanfäden in Längsrichtung unter Anwendung der Nähwirktechnik MALIWATT erhalten wurde. Die Nähwirktechnik MALIWATT ist in Malimo Nähwirktechnologie, Ploch, Böttcher, Scharch, VEB Fachbuchverlag Leipzig, 1978, 1. Auflage beschrieben. Diese Bindenlage ist auf beiden Oberflächen mit einem kohäsiv haftenden Klebemittel auf Basis Polyisopren-Kautschuk beschichtet, um den gewünschten kohäsiven Hafteffekt zu erreichen. Die Binde ist dabei wie folgt aufgebaut:
60 % Polypropylen ,12 % Elastan, 28 % IR-Kautschuk Grund-Vlies: PP-Spunbondvlies, 35 g/qm, thermisch geprägt Nähfaden 133 dtex Elastan (DORLASTAN, BAYER) Nähfadendichte 45 Fäden je 10 cm Breite Nähfaden-Stichlänge, Bindung 3 mm, offene Franse Flächengewicht gedehnt 59 g/qm Elastizität in Längsrichtung (Kettrichtung) Dehnbarkeit / Rückzug nach DIN 61632 160 % / 99 % Haftkraft Seite A/B 60 cN/cm.

Die Erfindung betrifft daher auch einen Kombinationsverband aus der beschriebenen Kompressionsbinde mit erster und zweiter Vliesschicht, die insbesondere einen Arbeitsdruck für die Kompressionstherapie bereitstellt, und einer weiteren Binde, die insbesondere als Langzugbinde für den Ruhedruck ausgebildet ist.

Die Einteilung in die Kategorien Kurz-, Mittel- oder Langzugbinde erfolgt je nach Dehnbarkeit und kann z.B. P. Asmussen, B. Söllner, Kompressionstherapie Prinzipien und Praxis, Verlag Urban & Fischer in Elsevier, 2004 auf Seite 121 entnommen werden. Die Dehnbarkeiten werden dabei nach DIN 61632 bestimmt.

Die Erfindung wird im Folgenden anhand einer Zeichnung näher beschrieben. Weitere Vorteile und Merkmale der Erfindung ergeben sich darüber hinaus aus den übrigen Anmeldungsunterlagen.

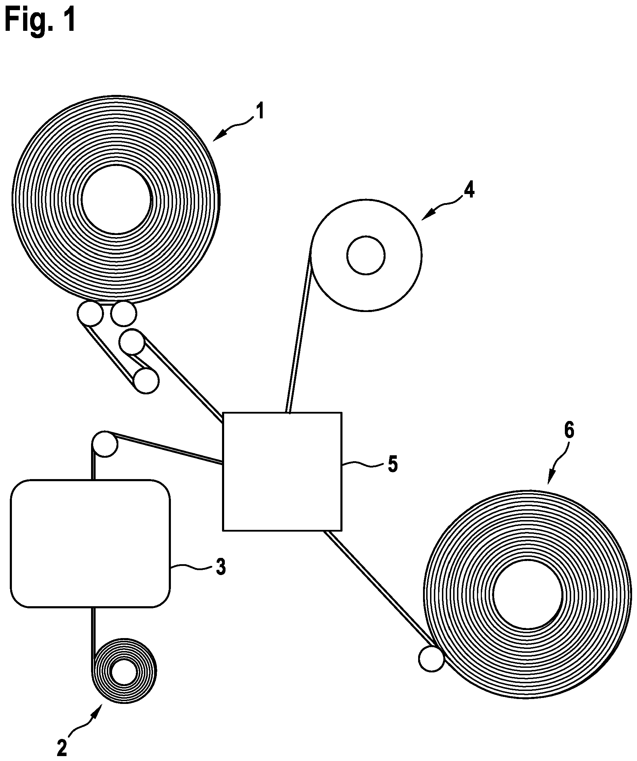

In der Zeichnung zeigt Figur 1 ein Herstellverfahren einer derartigen Kompressionsbinde.

In Figur 1 ist dargestellt, dass eine erste Vliesschicht 2, die mit superabsorbierenden Fasern versehen ist und eine zweite Vliesschicht 1 jeweils als Rollenmaterial zugeführt werden. Die erste Vliesschicht 2 kann dabei aus einem mit superabsorbierenden Fasern vorkonfektionierten Vlies bestehen oder vollständig aus superabsorbierenden Fasern oder es kann in der Einrichtung 3 ein Vliesmaterial mit superabsorbierenden Fasern belegt werden. Die beiden Vliesmaterialien 1 und 2 werden im bevorzugten Fall in einem Nähwirkverfahren mittels eine Kettenwirkmaschine, die mit dem Bezugszeichen 5 versehen ist, miteinander verbunden werden, wobei die Elastifizierungsmittel 4, die hier als Elastanfäden ausgebildet sind und als Kettfäden dienen, der Kettwirkmaschine 5 zugeführt werden. Das Nähwirkverfahren arbeitet dabei mit einem Haken, mittels dem die Schichten mit dem elastischen Nähfäden übernäht werden. Bei dem Nähfaden handelt es sich um einen elastisch vorgedehnten Faden, der mit einer vorgegebenen Stichlänge und einer vorgegebenen Nähfadenspannung eingebracht wird, und so nach Entspannung des elastischen Materials zu einem Zusammenziehen des Lagenverbundes (Flächengebildes) führt.

Alternativ können auch andere Elastifizierungsmittel eingesetzt und zugeführt werden.

Sofern keine Nähwirkverfahren eingesetzt wird, kann eine andere Einrichtung 5 eingesetzt werden, in der die Lagen miteinander verbunden und elastifiziert werden.

Das miteinander insbesondere über den elastischen Nähfaden verbundene Material wird dann auf eine Rolle 6 aufgewickelt, wobei gegebenenfalls vorab eine Konfektionierung in Längsrichtung zu Binden erfolgen kann.

Die so ausgebildeten Kompressionsbinden entsprechen in ihrer Charakteristik einer Kurzzugbinde, die zunächst annähernd keine Krafterhöhung bei Dehnung aufweist, um dann einen sprunghaften Anstieg und damit eine Dehnungsschwelle zu realisieren, wobei die Dehnungsschwelle hier im Bereich des optimalen Therapiedrucks liegt, so dass bei maximaler Dehnung der Kompressionsbinde gleichzeitig bei Anlegen unter dieser Vorspannung der optimale Therapiedruck für die Kompressionstherapie eingestellt werden kann.

Durch die in der ersten Vliesschicht enthaltenen superabsorbierenden Fasern können Wundexsudate gebunden werden und von der Wunde weggeleitet werden. Hierdurch kann die Wundheilung positiv beeinflusst werden, da Enzyme, die sich negativ auf die Heilung auswirken können, so aus der Wunde entfernt werden.

## Patentansprüche

1. Kompressionsbinde umfassend eine erste Vliesschicht (1) und mindestens eine flächig mit der ersten Vliesschicht verbundene zweite Vliesschicht (2), wobei die beiden Vliesschichten (1,2) mittels Nähwirkverfahren über einen elastischen Nähfaden (4) miteinander verbunden sind, **dadurch gekennzeichnet, dass** in der ersten Vliesschicht superabsorbierende Fasern vorgesehen sind.

2. Kompressionsbinde nach Anspruch 1, **dadurch gekennzeichnet, dass** die erste Vliesschicht (1) im angelegten Zustand auf einen Träger der Kompressionsbinde zu gerichtet ist und insbesondere als Polsterschicht ausgebildet ist.

3. Kompressionsbinde nach einem der vorangehenden Ansprüche, **dadurch gekennzeichnet, dass** die erste Vliesschicht (1) ein Thermofusionsvlies ist.

4. Kompressionsbinde nach einem der vorangehenden Ansprüche, **dadurch gekennzeichnet, dass** die zweite Vliesschicht (2) ein Thermobondvlies ist.

5. Kompressionsbinde nach einem der vorangehenden Ansprüche, **dadurch gekennzeichnet, dass** das Material einer oder beider der Schichten (1, 2) unelastisch ist.

6. Kompressionsbinde nach einem der vorangehenden Ansprüche, **dadurch gekennzeichnet, dass** die Dicke der ersten Vliesschicht (1) 0,6-1,2 mm beträgt.

7. Kompressionsbinde nach einem der vorhergehenden Ansprüche, **dadurch gekennzeichnet, dass** das Vliesmaterial der ersten und/oder zweiten Vliesschicht (2) Polyester umfasst, insbesondere daraus besteht.

8. Kompressionsbinde nach einem der vorhergehenden Ansprüche, **dadurch gekennzeichnet, dass** die superabsorbierenden Fasern aus Polyacrylat bestehen.

9. Kompressionsbinde nach einem der vorangehenden Ansprüche 5-8, **dadurch gekennzeichnet, dass** die elastischen Nähfäden (4) aus einer Gruppe von BaumwollSpinnkreppfäden, Baumwoll-Zwirnkreppfäden, texturierten Polyamidgarnen, texturierten Polyestergarnen, Gummifäden oder Polyurethan-Elastanfäden oder einer Kombination hieraus ausgewählt werden.

10. Kompressionsbinde nach einem der vorangehenden Ansprüche, **dadurch gekennzeichnet, dass** der Kompressionsbinde eine Dehnungsschwelle aufweist, die die maximale Dehnbarkeit anzeigt.

11. Kompressionsbinde nach einem der vorangehenden Ansprüche, **dadurch gekennzeichnet, dass** die maximale Dehnbarkeit einem therapeutisch vorgegebenen Anlegedruck der Kompressionsbinde an eine Gliedmaße eines Trägers entspricht.

12. Kompressionsbinde nach einem der vorangehenden Ansprüche, **dadurch gekennzeichnet, dass** eine vom Träger weg weisende Seite der Kompressionsbinde eine kohäsive Beschichtung aufweist.

13. Kompressionsbindezusammenstellung umfassend eine erste Kompressionsbinde nach einem der vorangehenden Ansprüche sowie eine zweite Kompressionsbinde, die insbesondere über die erste Kompressionsbinde anlegbar ist.

14. Kompressionsbindezusammenstellung nach Anspruch 13, **dadurch gekennzeichnet, dass** die erste Kompressionsbinde einen Arbeitsdruck und die zweite Kompressionsbinde einen Ruhedruck im angelegten Zustand auf eine Gliedmaße aufbringt.

## Claims

1. Compression bandage comprising a first nonwoven layer (1) and at least a second nonwoven layer (2) which is connected to the first nonwoven layer in a planar manner, the two nonwoven layers (1,2) being interconnected by means of a stitch-bonding process via an elastic sewing thread (4), **characterized in that** superabsorbent fibers are provided in the first nonwoven layer.

2. Compression bandage according to claim 1, **characterized in that** the first nonwoven layer (1) is directed towards a wearer of the compression bandage in the applied state and is designed in particular as a padding layer.

3. Compression bandage according to any one of the preceding claims, **characterized in that** the first nonwoven layer (1) is a thermofusion nonwoven.

4. Compression bandage according to any one of the preceding claims, **characterized in that** the second nonwoven layer (2) is a thermobond nonwoven.

5. Compression bandage according to any one of the preceding claims, **characterized in that** the material of one or both of the layers (1, 2) is inelastic.

6. Compression bandage according to any one of the preceding claims, **characterized in that** the thickness of the first nonwoven layer (1) is 0.6-1.2 mm.

7. Compression bandage according to any one of the preceding claims, **characterized in that** the nonwoven material of the first and/or second nonwoven layer (2) comprises polyester, in particular consists thereof.

8. Compression bandage according to any one of the preceding claims, **characterized in that** the superabsorbent fibers consist of polyacrylate.

9. Compression bandage according to any one of the preceding claims 5-8, **characterized in that** the elastic sewing threads (4) are selected from a group of cotton spun crepe threads, cotton twisted crepe threads, textured polyamide yarns, textured polyester yarns, rubber threads or polyurethane elastane threads or a combination thereof.

10. Compression bandage according to any one of the preceding claims, **characterized in that** the compression bandage has a stretch threshold that indicates the maximum stretchability.

11. Compression bandage according to any one of the preceding claims, **characterized in that** the maximum stretchability corresponds to a therapeutically predetermined application pressure of the compression bandage on a limb of a wearer.

12. Compression bandage according to any one of the preceding claims, **characterized in that** a side of the compression bandage facing away from the wearer has a cohesive coating.

13. Compression bandage assembly comprising a first compression bandage according to any one of the preceding claims and a second compression bandage which can be applied in particular over the first compression bandage.

14. Compression bandage assembly according to claim 13, **characterized in that** the first compression bandage applies a working pressure and the second compression bandage applies a resting pressure to a limb in the applied state.

## Revendications

1. Bande de compression comprenant une première couche de non-tissé (1) et au moins une deuxième couche de non-tissé (2) reliée à plat à la première couche de non-tissé, dans laquelle les deux couches de non-tissé (1, 2) sont reliées entre elles par un fil de couture élastique (4) au moyen d'un procédé de couture-tricotage, **caractérisée en ce que** des fibres super absorbantes sont prévues dans la première couche de non-tissé.

2. Bande de compression selon la revendication 1, **caractérisée en ce que** la première couche de non-tissé (1) est dirigée vers un support de la bande de compression à l'état posé et est réalisée en particulier comme une couche de rembourrage.

3. Bande de compression selon l'une quelconque des revendications précédentes, **caractérisée en ce que** la première couche de non-tissé (1) est un non-tissé pour thermofusion.

4. Bande de compression selon l'une quelconque des revendications précédentes, **caractérisée en ce que** la deuxième couche de non-tissé (2) est un non-tissé pour thermoliage.

5. Bande de compression selon l'une quelconque des revendications précédentes, **caractérisée en ce que** le matériau d'une ou des deux des couches (1, 2) est non élastique.

6. Bande de compression selon l'une quelconque des revendications précédentes, **caractérisée en ce que** l'épaisseur de la première couche de non-tissé (1) est de 0,6 à 1,2 mm.

7. Bande de compression selon l'une quelconque des revendications précédentes, **caractérisée en ce que** le matériau non tissé de la première et/ou de la deuxième couche de non-tissé (2) comprend du polyester, en particulier en est constitué.

8. Bande de compression selon l'une quelconque des revendications précédentes, **caractérisée en ce que** les fibres superabsorbantes sont constituées de polyacrylate.

9. Bande de compression selon l'une quelconque des revendications précédentes 5-8, **caractérisée en ce que** les fils de couture élastiques (4) sont choisis dans un groupe de fils de crêpe tissé de coton, fils de crêpe de coton retors, de fils de polyamide texturés, de fils de polyester texturés, de fils de caoutchouc ou de fils élasthannes de polyuréthane, ou d'une combinaison de ceux-ci.

10. Bande de compression selon l'une quelconque des revendications précédentes, **caractérisée en ce que** la bande de compression présente un seuil d'extension indiquant l'extensibilité maximale.

11. Bande de compression selon l'une quelconque des revendications précédentes, **caractérisée en ce que** l'extensibilité maximale correspond à une pression d'application de la bande de compression sur un membre d'un porteur, prédéterminée de manière thérapeutique.

12. Bande de compression selon l'une quelconque des revendications précédentes, **caractérisée en ce qu'**un côté de la bande de compression orienté à l'opposé du porteur présente un revêtement cohésif.

13. Combinaison de bande de compression comprenant une première bande de compression selon l'une quelconque des revendications précédentes ainsi qu'une deuxième bande de compression qui peut être posée en particulier sur la première bande de compression.

14. Combinaison de bande de compression selon la revendication 13, **caractérisée en ce que** la première bande de compression applique une pression de travail et la deuxième bande de compression une pression de repos sur un membre à l'état posé.
